# EUROPEAN PATENT SPECIFICATION

(11) **EP 1 654 352 B1**
(45) Date of publication and mention of the grant of the patent: **16.01.2008**
(21) Application number: 04743484.0
(22) Date of filing: 19.07.2004
(51) Int. Cl.: C12N 15/64, C12R 1/91, A61K 48/00

(54) **REVERSIBLY IMMORTALISED OLFACTORY ENSHEATHING GLIA AND THEIR USE TO PROMOTE NEURONAL REGENERATION**
REVERSIBEL IMMORTALIZIERTE, OLFAKTORISCHE NERVEN EINSCHLIESSENDE GLIA ZELLEN UND DEREN VERWENDUNG ZUR FÖRDERUNG DER NERVENREGENERIERUNG
NEVROGLIES ENVELOPPANTES OLFACTIVES IMMORTALISEES DE MANIERE REVERSIBLE ET LEUR UTILISATION DANS LA PROMOTION DE LA REGENERATION NEURONALE

(30) Priority: 18.07.2003 GB 0316882
(43) Date of publication of application: 10.05.2006
(73) Proprietor: CONSEJO SUPERIOR DE INVESTIGACIONES CIENTIFICAS, E-28006 Madrid (ES); Universidad Autónoma de Madrid, 28049 Madrid (ES)
(72) Inventor: MORENO FLORES, Maria, Teresa Centro de Biologia, Autónoma de Madrid Facultad de Ciencias Campus de Cantoblanco 28049 Madrid (ES); MARTIN BERMEJO, Maria, Jesús Centro de Biologia, Autónoma de Madrid Facultad de Ciencias Campus de Cantoblanco 28049 Madrid (ES); AVILA DE GRADO, Jesús Centro de Biologia Molecular, de Madrid Facultad de Ciencias Campus de Cantoblanco 28049 Madrid (ES); WANDOSELL, Jurado, Francisco Centro de Biologia, Autónoma de Madrid Facultad de Ciencias 28049 Madrid (ES); DIAZ NIDO, Javier Centro de Biologia Molecular, Madrid Facultad de Ciencias Campus de Cantoblanco 28049 Madrid (ES); LIM, Filip Centro de Biologia Molecular "Severo, Facultad de Ciencias Campus de Cantoblanco 28049 Madrid (ES); PASTRANA IZQUIERDO, Erika Centro de Biologia, Autónoma de Madrid Facultad de Ciencias Campus de Cantoblanco 28049 Madrid (ES)
(74) Representative: Ruffles, Graham Keith
(86) International application number: PCT/GB2004/003149
(87) International publication number: WO 2005/012513

(56) References cited:
- WO-A-02/088337
- US-A- 5 629 159
- MORENO-FLORES M TERESA ET AL: "Immortalized olfactory ensheathing glia promote axonal regeneration of rat retinal ganglion neurons." JOURNAL OF NEUROCHEMISTRY, vol. 85, no. 4, May 2003 (2003-05), pages 861-871, XP002302026 ISSN: 0022-3042 cited in the application
- PAILLARD F: "Reversible cell immortalization with the Cre-lox system." HUMAN GENE THERAPY. 1 JUL 1999, vol. 10, no. 10, 1 July 1999 (1999-07-01), pages 1597-1598, XP002302027 ISSN: 1043-0342
- SALMON P ET AL: "REVERSIBLE IMMORTALIZATION OF HUMAN PRIMARY CELLS BY LENTIVECTOR-MEDIATED TRANSFER OF SPECIFIC GENES" MOLECULAR THERAPY, ACADEMIC PRESS, SAN DIEGO, CA,, US, vol. 2, no. 4, October 2000 (2000-10), pages 404-414, XP001028604 ISSN: 1525-0016 cited in the application
- NALDINI ET AL: "Efficient transfer, integration, and sustained long-term expression of the transgene in adult rat brains injected with a lentiviral vector" PROCEEDINGS OF THE NATIONAL ACADEMY OF SCIENCES OF USA, NATIONAL ACADEMY OF SCIENCE. WASHINGTON, US, vol. 93, no. 21, 15 October 1996 (1996-10-15), pages 11382-11388, XP002114690 ISSN: 0027-8424 cited in the application
- WESTERMAN K A ET AL: "REVERSIBLE IMMORTALIZATION OF MAMMALIAN CELLS MEDIATED BY RETROVIRAL TRANSFER AND SITE-SPECIFIC RECOMBINATION" PROCEEDINGS OF THE NATIONAL ACADEMY OF SCIENCES OF USA, NATIONAL ACADEMY OF SCIENCE. WASHINGTON, US, vol. 93, no. 17, 20 August 1996 (1996-08-20), pages 8971-8976, XP000674657 ISSN: 0027-8424 cited in the application
- SANTOS-BENITO FERNANDO F ET AL: "Olfactory ensheathing glia transplantation: a therapy to promote repair in the mammalian central nervous system." THE ANATOMICAL RECORD. MAR 2003, vol. 271B, no. 1, March 2003 (2003-03), pages 77-85, XP002302028 ISSN: 0003-276X
- RAMON-CUETO A ET AL: "OLFACTORY ENSHEATHING GLIA: PROPERTIES AND FUNCTION" BRAIN RESEARCH BULLETIN, ELSEVIER SCIENCE LTD, OXFORD, GB, vol. 46, no. 3, June 1998 (1998-06), pages 175-187, XP001157028 ISSN: 0361-9230 cited in the application

## Description

### FIELD OF THE INVENTION

This invention relates to neuronal regeneration. In particular, the invention provides a specific type of cells, namely, olfactory ensheathing glia (OEG) reversibly immortalised and grown in culture, which are functional and safe for use in transplantation. The invention has particular application in transplantations directed to neural regions, for example brain, spine and/or peripheral nerves, of a human to assist recovery of acute and chronic nerve damage following surgery or trauma.

### BACKGROUND OF THE INVENTION

### Olfactory ensheathing glia (OEG)

From the studies of Ramón y Cajal it is known that the ability of adult central nervous system (CNS) neurons to regenerate is extremely limited. In contrast, neurons from the peripheral nervous system (PNS) have a notable capacity of regeneration, which may be due, among other factors, to the particular characteristics of the Schwann cells (SC) which ensheath PNS axons. This fact has stimulated the use of peripheral nerve and SC grafts to foster regeneration in CNS, with promising results (reviewed in Jones LL, et al. "Neurotrophic factors, cellular bridges and gene therapy for spinal cord injury", J. Physiol 2001; 533:83-89). Nevertheless, the incomplete CNS regeneration achieved with SC makes necessary the search for more effective regeneration mediators.

During the last years the use of OEG cells for CNS regeneration have received plenty of attention, due to their special properties. The olfactory system has remarkable distinctive properties within the adult mammalian central nervous system. Throughout the whole life of an organism, olfactory sensory neurons are renewed from progenitor cells present at the olfactory neuroepithelium. New-born sensory neurons extend new axons that grow and enter in the CNS to make their appropriate connections in the glomerular layer of the olfactory bulbs. These olfactory axons are surrounded by a special type of glial cells, called olfactory ensheathing glia (OEG).

*In vivo* OEG cells express several markers that have been used for their identification after isolation and culture (see Ramon-Cueto A, Avila J. "Olfactory ensheathing glia: properties and function", Brain Res. Bull. 1998; 46:175-187 and references therein). OEG cells share some properties with Schwann cells and astrocytes, although they present a distinct pattern of markers and properties which allows to classify them as a different class of glial cells.

Two OEG cells variants have been identified in cultures. In serum-free medium Barnett and co-workers (Franceschini IA, Barnett SC. "Low-affinity NGF-receptor and EN-CAM expression define two types of olfactory nerve ensheathing cells that share a common lineage". Dev.Biol. 1996; 173:327-343) have identified two extreme morphological types in the OEG cells cultures, an astrocyte-like flat cell, which express GFAP (fibrous staining) and PSA-NCAM, and is negative for p75-NGFr, and a second type, a Schwann-like spindle cell, expressing p75-NGFr, with diffuse staining for GFAP and negative for PSA-NCAM. All the intermediate phenotypes are possible and the observation of these phenotypes even in an established clonally cell line supports the view that they can derive from a common precursor. In our OEG cells cultures, from adult rats and human donors, we have also detected both morphological types of cells and the intermediate phenotypes.

From the data obtained from our lab and other labs, it is obvious that OEGs are clearly distinct from astrocytes or Schwann cells, independently of the age of the murine or human donor.

The capacity of OEG cells to promote neurite outgrowth can be used to establish neuritogenesis and/or regeneration models in culture. Neurite outgrowth in culture may simply indicate neuritogenesis when using embryonic or neonatal neurons. However, neurite extension from adult CNS neurons can be considered as a culture model of CNS regeneration. Thus, Wigley and Berry have established a co-culture model using adult retinal ganglion cells (from now, RGC) on a monolayer of glial cells. Adult RGC cultured on a confluent monolayer of neonatal cortical astrocytes are able to re-grow neurites over long distances (Wigley CB, Berry M. "Regeneration of adult rat retinal ganglion cell processes in monolayer culture: comparisons between cultures of adult and neonatal neurons", Brain Res. 1988; 470:85-98). In a recent study, Wigley and co-workers, using the same model, compared monolayer of adult rat OEG, with neonatal astrocytes and SC. They demonstrated that adult rat OEG cells were the best substrate for neurite regeneration of adult RGC in culture, and this effect mainly depended on intercellular contact and calcium. Cellular surface molecules (factors for adhesion, axonal guiding, etc.) are important for adult RGC as demonstrated by Sonigra and co-workers (Sonigra RJ, Brighton PC, Jacoby J, Hall S, Wigley CB. "Adult rat olfactory nerve ensheathing cells are effective promoters of adult central nervous system neurite outgrowth in coculture". Glia 1999; 25:256-269), but their exact identity has to be defined.

Initially regeneration in CNS has been achieved by the use of PNS Schwann cells (SC) grafts. CNS re-growing axons are able to penetrate into this permissive environment but the problem is the low efficiency of re-entry in CNS tissue distal to the lesion. Previous experiments indicated that OEG cells were able to mediate re-entry of peripheral sensory axons in spinal cord CNS environment after rizhotomy. Re-entry of regenerating axons into the CNS has also been observed after a complete transection of the spinal cord, when a bridge tube with SC and OEG cells was placed. The presence of OEG cells decreased the formation of glial scar and constituted an axonal passageway at the interface graft-host. This method provided a way of increasing the efficiency of re-entry of regenerating axons in the CNS host tissue. Using the model of rat spinal cord complete transection, an extraordinary grade of CNS regeneration affecting to several tracts in the spinal cord, has also been demonstrated, with accompanying functional recovery as measured by behavioural tests ( Ramon-Cueto A, et al. "Functional recovery of paraplegic rats and motor axon regeneration in their spinal cords by olfactory ensheathing glia", Neuron 2000; 25:425-435). OEG cells seem to be able to migrate in the host CNS tissue and to go along with the growing axons thus providing a micro-environment favourable for CNS regeneration.

All these data support the view that OEG cells are functionally different from the other neural cells, such as astrocytes or Schwan cells.

One of the most important requisites for the use of these cells is a complete understanding of their growth requirements. Certain mitogens for OEG cells were initially characterised like factors present in astrocyte conditioned medium (ACM), that do not bind to heparin and can be inhibited with antibodies against neuregulin-1 (NRG-1). Moreover, semipurified bovine pituitary extract, which is a crude source of several glial mitogens including GGF a NRG-1, is active for OEG.

Whereas experiments performed in rodents suggest that OEG cells grafts favour functional recovery after spinal cord injuries, these studies have to be extended to primates, where the supraspinal control of motor functions is fundamental to asses functional recovery. Of the utmost importance in this respect is the availability of an accessible and non-limited source of human OEG cells for grafting into patients. We have defined conditions for prolonged subculture of adult rat OEG cells using a specific mixture of trophic factors, biochemical compounds and medium. This combination also allows the growth of the human OEG cells. However, after long-term culture OEG cells lose their ability to support axonal regeneration from adult CNS neurons although they keep the ability to support axonal extension from young neurons. Thus long-term culture of OEG cells seems not to be an alternative to obtain a sufficient population for grafting.

WO 02/088337 discloses a method for obtaining high amounts of OEG cells which comprises infecting primary OEG cells with retroviral vectors said vectors comprising the telomerase catalytic sub-unit Tert, in order to obtain modified OEG cells. However, nothing is mentioned about the ability of said cells to support axonal regeneration from adult CNS neurons.

The generation of immortalised non-tumourigenic clonal cell lines which retain the axonal regeneration-promoting properties of primary OEG cells cultures is an alternative to have unlimited amount of OEGs. Cloned rat OEG cell lines have been developed by immortalization using the SV40 large T antigen expressed from a constitutive cellular promoter (Moreno-Flores MT,et al. "Immortalised olfactory ensheathing glia promote axonal regeneration of rat retinal ganglion neurons", J. Neurochem. 2003; 85:861-871). Some of these immortalised rat OEGs were found comparable to primary rat OEG cells and SC in the promotion of axonal regeneration of mature RGN. However, the continued presence of the oncogene (in this case a gene encoding SV40 large T antigen) in these cells is of concern, inasmuch as it may increase the risk of malignant transformation following transplantation.

Thus, there is still a need for an accessible and non-limited source of human OEG cells which retain the axonal regeneration-promoting properties and at the same time avoid the generation of tumours.

### SUMMARY OF THE INVENTION

The object of our invention is based on the capacity of OEG cells to promote neuronal axonal regeneration *in vitro* and *in vivo,* an ability not shared, so far, by any other cell type primary or genetically modified, at this extent. We have found a way to obtain by reversible genetic modification and selection unlimited amounts of OEG cells which keep their axonal regeneration capacity.

In the course of our investigations we have found a procedure to reverse immortalise primary cells and obtain immortalised clonal cell lines of OEGs from human donors. We have tested that all of these cell lines closely resemble primary OEGs in their molecular markers. Significantly, we have found that some of these cell lines maintained their ability to promote axonal regeneration from adult neurons, even when the immortalization process is reversed.

According to one aspect of the instant invention, a functional reversibly-immortalised or reverse-immortalised human olfactory ensheathing glia (OEG) cell line is provided. Said cell line may be used in a method of therapy, such as in a method of promoting neuronal regeneration by transplanting said cells into regions of the injury in the central and/or peripheral nervous system.

In another aspect of the invention, a method of making a population of functional human OEG cells for transplantation into a patient is provided. The method comprises: (a) providing a sample of primary OEG; (b) immortalizing the OEG cells by transforming the OEG cells with a vector comprising a removable DNA segment containing an oncogene (or combination of oncogenes), thereby producing immortalised OEG cells; (c) growing the immortalised OEG cells; (d) selecting those OEG clonal cell lines which maintain the functional properties of parent OEG cells (i.e., the ability to trigger axonal regeneration from adult CNS neurons), and (e) removing the oncogene (or combination of oncogenes) from the immortalised OEG cells, the removal resulting in the production of the population of functional OEG cells for transplantation into the patient. Preferably, the OEG cells are obtained from a human donor and the oncogene is any gene able to bypass entry into senescence including those encoding SV40 large T antigen, telomerase catalytic subunit, Bmi-1 protein or any combination of these oncogenes.

Another aspect of the invention provides a pharmaceutical composition comprising said functional human olfactory ensheathing glia (OEG) cell, and a pharmaceutically acceptable carrier. In a preferred embodiment the cell is a reverse-immortilized OEG cell.

The oncogene (or combination of oncogenes) is made removable preferably by flanking it with recombinase target sites, and the removing is accomplished by introducing into the immortalised cells a gene that is expressed to produce a recombinase that specifically recognizes the recombinase target sites. Preferably, the recombinase is Cre recombinase and the recombinase target sites are loxP sites.

In preferred embodiments, the removable DNA segment further contains a suicide gene, which encodes a gene product that enables destruction of the immortalised cells by an exogenous agent if the removable DNA segment is not removed from the cells. The suicide gene preferably is a gene encoding herpes simplex virus thymidine kinase, and the cells are destroyed by exposure to gancyclovir if the removable DNA segment is not removed from the cells.

Another aspect of the invention provides a method of making a population of functional human OEG cells for transplantation into a patient, which comprises: (a) providing a sample of primary OEG; (b) immortalizing the OEG cells by transforming the OEG cells with a vector comprising a removable DNA construct containing an oncogene (or combination of oncogenes), a selectable marker gene, and a gene encoding herpes simplex virus thymidine kinase, the genes together being flanked on either side by loxP sites; (c) growing the immortalised OEG; (d) selecting those OEG clonal cell lines which maintain the functional properties of parent OEG cells, and (e) reversing the immortalization of the OEG cells by removing the DNA construct from the immortalised OEG cells, the removing being accomplished by introducing into the immortalised OEG cells a gene encoding Cre recombinase to effect excision of the DNA construct at the loxP sites, the excision resulting in the production of the population of functional OEG cells for transplantation into the patient.

Populations of functional human OEG cells produced by the aforementioned methods are also provided, along with the use of human OEG cells produced by the aforementioned methods in the manufacture of a medicament for treating a patient for neuronal damage, comprising transplanting said OEG cells into neural regions, for example brain, spine and/or peripheral nerves, of damage or injury of a patient in a sufficient quantity of those OEG cells to repair said neuronal damages or injuries in the patient or to assist recovery of acute and chronic nerve damage following surgery or trauma.

According to another aspect of the invention, a reversibly immortalised human OEG cell is provided, which comprises a primary OEG cell transformed with a DNA construct comprising two recombinase target sites that flank an oncogene (or combination of oncogenes) which confers immortalization to the OEG cell, wherein the immortalization is reversible by excision of the DNA construct by cleavage at the recombinase target sites when the target sites are exposed to a recombinase that specifically recognizes the target sites. Preferably, the recombinase target sites are loxP sites and the immortalization is reversible by Cre recombinase cleavage at the loxP sites. The DNA construct can further include a selectable marker gene, and may further comprise a suicide gene, which encodes a gene product that enables destruction of the immortalised OEG cell by an exogenous agent if the DNA construct is not removed from the cells. Preferably, the suicide gene is a gene encoding herpes simplex virus thymidine kinase, and the exogenous agent is gancyclovir.

In one embodiment, a reversibly immortalised human OEG cell line is provided.

According to another aspect of the invention, a reverse-immortalised human OEG cell that is functional upon transplantation into a patient is provided, which is produced by exposing the DNA construct within the above-described immortalised OEG cell to a recombinase that excises the DNA construct by cleavage at the recombinase target sites. The use of above- described immortalised OEG cell in the manufacture of a medicament for treating a patient for neuronal damage is also provided, wherein the medicament comprises a sufficient quantity of said reverse-immortalised OEG cell for transplanting into the injured region of a patient in order to promote neuronal regeneration in the patient.

### BRIEF DESCRIPTION OF THE DRAWINGS

**Figure 1** shows the general morphology of human OEGs cells after dissociation and cultured for one week (DN 1) or after five consecutives passages (DIV 2).
**Figure 2** shows the immunofluorescence of human OEGs stained with antibodies against glial markers, such as Neuroligin, APP (22C11), S100 and GFAP.
**Figure 3** shows the expression of members of the EGF-R family. Cells extract obtained from human OEGs in different medium were analysed by Western blot with antibodies against Erb B2, Erb B3, and Erb B4. As a loading control tubulin was used. The Figure contains the data from a similar experiment with parallel culture medium from established cell lines from rat OEGs.
**Figure 4** shows the axonal regeneration capacity of rat retinal neurons stained with antibodies against neuronal proteins such as neuronal specific tubulin (334), axonal proteins such as MAP1B-SMI31 (SMI31) or MAP2 (305), or somato-dendritic protein MAP2 (514).
**Figure 5** shows a schematic representation of the lentiviral construct pLOX-Ttag-Ires-TK containing as oncogene the gene encoding SV40 large T antigen.
**Figure 6** shows a schematic representation of the lentiviral construct pLOX-HTERT-Ires-TK containing as oncogene the gene encoding the telomerase catalytic subunit.
**Figure 7** shows a schematic representation of the lentiviral construct pLOX-CWBmi1 containing as oncogene the gene encoding Bmi-1 protein.

### DETAILED DESCRIPTION OF THE INVENTION

Certain aspects of the present invention employ conventional molecular biology, microbiology, and recombinant DNA techniques that are well known in the art. See, e.g., Sambrook et al., "Molecular Cloning: A Laboratory Manual" (1989); "DNA Cloning: A Practical Approach", Volumes I and II (D. N. Glover ed. 1985); "Oligonucleotide Synthesis" (M. J. Gait ed. 1984); "Nucleic Acid Hybridization" [B. D. Hames & S. Higgins eds. (1985)]; "Transcription and Translation" [B. D. Hames & S. J. Higgins eds. (1984)]; "Animal Cell Culture" [R. I. Freshney, ed. (1986)]; "Immobilized Cells And Enzymes" [IRL Press, (1986)]; B. Perbal, "A Practical Guide To Molecular Cloning" (1984); or "Current Protocols in Molecular Biology", eds. Frederick M. Ausubel et al., John Wiley & Sons, 1999.

The term "nucleic acid construct" or "DNA construct" is sometimes used to refer to a coding sequence or sequences operably linked to appropriate regulatory sequences and inserted.into a vector for transforming a cell. This term may be used interchangeably with the term "transforming DNA". Such a nucleic acid construct may contain a coding sequence for a gene product of interest, along with a selectable marker gene and/or a reporter gene.

The term "operably linked" or "operably inserted" means here that the regulatory sequences necessary for expression of the coding sequence are placed in a nucleic acid molecule in the appropriate positions relative to the coding sequence so as to enable expression of the coding sequence. This same definition is sometimes applied to the arrangement other transcription control elements (e.g. enhancers) in an expression vector.

The term "selectable marker gene" refers to a gene encoding a product that, when expressed, confers a selectable phenotype such as antibiotic resistance on a transformed cell.

A "vector" is a replicon, such as plasmid, phage, cosmid, or virus to which another nucleic acid segment may be operably inserted so as to bring about the replication or expression of the segment. More specifically, the term "viral vector" refers to a virus that is able to transmit foreign or heterologous genetic information to a host. This foreign genetic information may be translated into a protein product, but this is not a necessary requirement for the foreign information.

The terms "immortalization" or "immortalised" refers to a cell, or a process for creating a cell, that will proliferate indefinitely in culture thus bypassing the entry into senescence. In the present invention, immortalization refers to a process by which a primary cell culture is transformed in a way that causes the cells to behave in some respects like a tumour cell; specifically, in the proliferative characteristics of tumour cells.

The term "selection" refers to a process by which immortalised OEG clonal cell lines are individually assayed for their ability to support neuronal survival and promote axonal regeneration from adult CNS neurons in order to choose exclusively those clonal cell lines maintaining this functional properties.

The term "reverse-immortalization" refers to a process by which cells are immortalised by a means enabling them to be returned to a non-immortalised state at a later time.

A "reversibly immortalised" cell is a cell that is presently in an immortalised state, but can be returned to a non-immortalised state at a later time, utilizing the reverse-immortalization process described herein.

A "reverse-immortalised" cell is a cell that has been subjected to the entire process of reverse-immortalization, and now exists in a non-immortalised state.

The term "functional" as used herein, means that the reverse-immortalised OEG cells are able to trigger axonal regeneration from adult CNS neurons *in vitro* in a co-culture system and therefore still have the capacity of promoting neuronal regeneration *in vivo* upon transplanting into the neural injured region of a patient.

The term "suicide gene" refers to a gene that confers a lethality phenotype to cells which are reversibly immortalised. In more common terms, the "suicide gene" can be thought of as a negative selectable marker gene. Expression of its gene product enables the cell to be killed, i.e., by treatment of the cell with an exogenous agent such as an antibiotic or antiviral agent.

The term "recombinase/recombinase target" refers to pairs of interacting molecules, one being a recombinase enzyme and the other being a DNA site specifically recognized and cleaved by that recombinase enzyme. The recombinase/recombinase target are paired by virtue of the specific interaction between the two, i.e., binding of the recombinase to its cognate DNA binding sequence, and cleavage of the DNA at that site.

The term "neuronal damage" as used herein refers to any injury leading to any functional neurological disability as a consequence of neuronal cell death, neuronal tract disorganization, axon degeneration or synapse elimination resulting from any acute or chronic traumatic lesion or any degenerative disease condition. Promotion of neuronal axonal regeneration may thus facilitate the repair of surviving neuronal circuits in a variety of situations including traumatic lesions of the brain and spinal cord, axonal degenerative diseases including multiple sclerosis and neuronal degenerative diseases including among other Alzheimer's, Parkinson, Huntington, motoneuron diseases and spinocerebellar ataxias.

In accordance with the present invention, a means is now available for minimizing or eliminating the risk of malignant transformation of transplanted human which have been produced by immortalization of primary human OEG cells and expansion in cell culture. The inventors have reversibly immortalised human OEG cells using a recombinant virus, such as a lentivirus, containing an oncogene or combination of oncogenes capable of inducing tumourigenic growth, flanked by recombinase target sites. Excision of the oncogene or combination of oncogenes from the immortalised cells is accomplished by site-specific recombination following introduction into the cells of a gene encoding the recombinase that specifically recognizes the recombinase target sites. After site-specific recombination and oncogene excision, cell proliferation stops and the cells develop the characteristics of primary human OEG cells. Moreover, the cells possess minimal oncogenic potential.

A significant feature of the reverse-immortalised human OEG cells described above is that the risk of their malignant transformation in transplant patients is greatly reduced. The safe use of these cells for OEG transplantation has been even further augmented in accordance with the invention, by the addition of a "suicide" gene to the initial viral construct used to immortalise the cells. In an exemplary embodiment, the suicide gene is a herpes simplex virus thymidine kinase (HSV-tk) gene, incorporated into the lentiviral vector between the two loxP sites. If the oncogene (or combination of oncogenes) is successfully excised by the Cre recombinase via the mechanism described above, the HSV-tk gene also is excised. If it is not excised, the cell can be destroyed by treatment with gancyclovir, an antiviral agent that targets the HSV-tk gene product.

Thus, the reversibly immortalise human OEG cells of the invention are human OEG cells that comprise a heterologous DNA construct comprising a selectable marker gene and an oncogene or combination of oncogenes that enables the cells to proliferate in culture, the selectable marker gene and the oncogene (or oncogenes) together being flanked by DNA binding sites for a recombinase. Optionally, the DNA segment further comprises a "suicide" gene, also disposed within the recombinase binding sites. The invention is practiced by transforming the primary human OEG cells with the DNA construct, culturing the transformed OEG cells under standard conditions suitable to expand the population of transformed OEG cells, then exposing the transformed OEG cells to the recombinase that recognizes the binding sites on the DNA construct (e.g. by infecting the transformed OEG cells with a viral vector containing a gene encoding the recombinase). If the DNA construct also contains a "suicide" gene, the OEG cells are further subjected to the conditions that will kill any cells still containing the DNA construct, following treatment with the recombinase.

Methods for obtaining and initiating cultures of primary human OEG cells are well known in the art. For example, primary OEG can be obtained from the patient or donor either form the olfactory bulb, which entails the obvious difficulties and risks, or from the olfactory mucosa lamina propia. With the invention we solve the problem of obtaining enough OEG cells for transplantation exclusively from the limited supply of the olfactory mucosa lamina propia. Human olfactory mucosa is relatively easy to biopsy via a simple endoscopic procedure or local anaesthesia (Feron et al. "new techniques for biopsy and culture of human olfactory epithelial neurons, Arch. Otorlaryngol. Head Neck Surg. 1998; 124: 861-866).

Any oncogene or combination of oncogenes may be used to reversibly immortalise primary OEG cells, and many of these are known in the art. The gene encoding SV40 large T antigen (SV40Tag), a known oncogene used to immortalise primary cells, is one of them, but others may be used. These include, but are not limited to, viral oncogenes as known in the art, and cellular oncogenes such as Bmi-1, telomerase catalytic subunit, c-myc, or genes encoding growth factor receptor among others. In a particular embodiment, the DNA segment comprises only one oncogene. In another particular embodiment the DNA segment comprises a combination of two or more oncogenes; in this case, the oncogenes may be in tandem or in any other suitable position.

The oncogene(s) can be delivered by a variety of methods, for example, within any vector suitable for delivering genetic material to cells. Lentivirus vectors are exemplified herein. Other suitable vectors include retrovirus vectors, specifically oncoretroviruses, as well as adeno-associated viruses and adenoviruses. Examples of lentivirus vectors include human immunodeficiency virus Type 1 (HIV-1), from which many suitable vectors have been developed. Other lentiviruses which are suitable for use as vectors include the primate lentivirus group including human immunodeficiency virus Type 2 (HIV-2), human immunodeficiency virus Type 3 (HIV-3), simian immunodeficiency virus (SIV), simian AIDS retrovirus (SRV-1), human T-cell lymphotropic virus Type 4 (HTLV4), as well as the bovine lentivirus, equine lentivirus, feline lentivirus, and ovine/caprine lentivirus groups. In addition to delivering the oncogene(s) by the methods indicated above, the oncogene(s) may be delivered by other means known in the art, for example, by electroporating into the cells in a manner similar to that taught in EP 235113.

Similarly, any selectable marker gene may be used in the DNA construct carrying the oncogene, and many of these are known in the art. Several selectable marker systems as well as vectors and other means for getting oncogenes into cells are described in "Current Protocols in Molecular Biology", eds. Frederick M. Ausubel et al., John Wiley & Sons, 2001.

In addition many examples of suitable "suicide" genes are known in the art. The HSV-tk gene is preferred for use, but others may be used. Many examples are set forth by Ausubel et al., 2001, *supra.*

In addition to the immortalizing oncogene or combination of oncogenes, selectable marker gene and the suicide gene, the DNA construct may comprise one or more desired genes, such as to promote growth.

The aforementioned genes may be operably linked to one or more 5' and/or 3' expression-controlling regions, as are known in the art. With reference to promoters, constitutive or inducible promoters may be utilized, also as is known in the art.

DNA recombinase systems suitable for use in the invention are also known in the art. The cre/lox system (Cre recombinase, LoxP binding sites) is preferred for use, but other systems can also be used, including, but are not limited to the FLP/FRT system from *Saccharomyces cerevisiae.* It will be understood that if the DNA construct contains target binding sites for a particular recombinase, it is that recombinase that is to be used in reversing the immortalization of the OEG cells.

US 5,629,159 describes a variety of DNA constructs exemplified for use in immortalization and dis-immortalization of pancreatic islet cells and neural cells. One or more of these variations may be adapted, in whole or in part, for reverse immortalization of OEG cells in accordance with the present invention, using the methods described herein.

A preferred embodiment of the invention comprises (1) immortalizing primary human OEG cells with a lentiviral vector containing the SV40Tag gene, the HSV-tk gene and a suitable selectable marker gene (e.g., neo or HSA, encoding the heat-stable antigen), flanked by loxP sites; (2) selecting transformants and growing them in culture; (3) reversing the immortalization by infecting the cells with a suitable vector, such as an adenovirus vector, carrying an expressible Cre recombinase gene to excise the oncogene; and, optionally, (4) destroying cells in which the oncogene was not successfully excised by treating the cells with gancyclovir. Vectors and systems of this type have been developed for reversible immortalization of various primary cells (Westerman & Leboulch, Proc. Natl. Acad. Sci. USA 93: 8971-8976, 1996), but have not been used for reverse immortalization of primary OEG cells. More importantly, prior to the present invention, it was unknown whether such a system could be used to produce OEG cells that would function *in vivo* following transplantation maintaining their axon regeneration properties and that would be of sufficiently low oncogenic potential to be safe for such use.

The preferred embodiments have been tested, as described in detail in the examples. Briefly, OEG cells from human olfactory bulbs were immortalised using a recombinant lentivirus containing the gene encoding SV40Tag flanked by loxP recombination target sites, in accordance with the protocol described by Dr. Trono's lab (Example 1). Excision of SV40Tag from immortalised cells could then be accomplished by site-specific recombination with Cre-recombinase. Cells immortalised with this recombinant virus expressed the oncogene [(SV40Tag in this case) or any other oncogen, or combination thereof], and they don't stop dividing. After excision of the gene encoding SV40Tag with Cre-recombinase, cells stopped growing and DNA synthesis fell and the cells become senescence after limited time period.

Taken together, these results demonstrate that the reverse-immortalised human OEG cells produced by the method described above function *in vivo* as OEG cells following transplantation. Moreover, the cells are of sufficiently low oncogenic potential to be safe for transplantation into patients in need of such treatment. Although similar reverse immortalization protocols have been employed for other cell types, the successful reversal of immortalization to yield *in vivo* functional human OEG cells was an unexpected result. In fact, human OEG cells obtained following reverse immortalization are capable of promoting neuronal regeneration (Example 2).

Thus, the present invention demonstrates that, by transducing primary human OEG cells with a recombinant virus incorporating an oncogene (or a combination of oncogenes), a suicide gene and a recombinase/recombinase target system, a well-differentiated, reversibly-immortalised non-tumourigenic human OEG cell line is generated. The successful generation of such a cell line would not have been predictable in advance of the results described in accordance with the present invention.

OEG cells transplantation holds great promise as an alternative to promote nervous system repair, neuronal regeneration, and treatment of neuronal damage, injuries or SNC lesions including traumatic lesions of the brain and spinal cord, axonal degenerative diseases including multiple sclerosis and neuronal degenerative diseases including among other Alzheimer's, Parkinson, Huntington, motoneuron diseases and spinocerebellar ataxias, for patients suffering said damages, injuries and lesions. A significant limitation to the development of this therapy related to the limited availability of OEG cells for transplantation has been overcome by the instant invention.

Therefore, in one aspect the present invention is also directed to the use of human OEG cells produced by the methods of the invention in the manufacture of a medicament for treating a human patient suffering from neuronal damage by treatment with an effective amount of a composition comprising the above defined reverse-immortilized OEG cells. The number of cells applied will depend of the size of the lesion and the condition of the patient being treated. The application will be usually by surgery to expose the site of the lesion so that the cells of the invention can be applied directly.

The treament can be autologous, thus using reverse-immortilized human OEG cells generated from OEG cells from the patient. This reduces the risk of adverse inmune reactions, although it has the disadvantage that some time is needed from the moment a sample is taken from the patient until sufficient reverse-immortalised cells are generated for the treatment. Alternatively, the treatment can be heterologous, using a bank of reversibly-immortilized or reverse-immortalised human OEG cells readily available. This bank of cells comprises a collection of reversibly-immortilized or reverse-immortalised human OEG cells prepared according to the herein described methos of the invention, and selecting those characterized by a large histocompatibility and effectiveness in promoting axonal regeneration. The availability of this collection of cells will reduce significantly the time needed for treatment, for example after a CNS injury, although the use of inmunosupresant compounds might be needed.

A bank of cells for transplantation comprising reversibly-immortilized or reverse immortalised human OEG cells is also an object of the invention.

In one embodiment the cell bank of the invention comprises a library or plurality of human reversibly-immortilized or reverse-immortalised OEG cell lines, each of which is homozygous for at least one or more critical antigen genes, i. e. , genes which encode histocompatibility antigens: Preferably each cell lines is homozygous for at least one MHC (Major Histocompatibility Complex) allele present in a human population, wherein each member of said plurality of cell lines is homozygous for a different set of MHCalleles relative to the remaining members of the plurality of OEG cell lines. Preferably, each member of the library is homozygous for a different combination of histocompatibility or MHC antigenalleles. The collection can be kept and distributed frozen to the sites of application. Using the methods of the present invention, a line of reversibly-immortilized or reverse-immortalised human OEG cells can be selected from a bank of such cells that are homozygous for one or more histocompatibility antigen alleles, in the case of MHC alleles that match an MHC allele of a patient in need of transplant.

A method of treatment using the human OEG cells of the invention can be combined with other therapies such as the application of neurotrophic or growth factors, pharmacological agents that mimic the action of neurotransmitters, anti-apoptotic agents, agents that interphere with inhibitors of growing axons, and physical rehabilitation and training. A polymer scaffold can be used to allow directed growth of the regenerating axons.

Pharmaceutical compositions comprising the cells of the invention are also envisaged. The cells may preferably be comprised in a pharmacologically suitable carrier such as a physiologically compatible vehicle, including, but not limited to, culture medium, such as Dulbecco's Modified Eagle's Medium, RPMI 1640, Fisher's, Iscove's, or McCoy's medium; or preferably, a solid, semisolid, gelatinous, or viscous support medium such as collagen, collagenglycosaminoglycan, fibrin, polyvinyl chloride, polyamino acids such as polylysine or polyomithine, hydrogels, agarose, dextran sulfate or silicone. The support medium may, in specific embodiments, comprise growth factors or other agents as decribed above.

If a solid, semisolid, or gelatinous support is used as a vehicle, cells may be introduced into a liquid phase of the vehicle which is subsequently treated such that it becomes more solid. In embodiments of the invention in which the vehicle has a solid structure, the vehicle may be molded into a shape which may conform to the shape of the lesion.

The following examples are provided to describe the invention in greater detail. They are intended to illustrate, not to limit, the invention.

### EXAMPLE 1

### Reverse immortalization of human primary OEG cells using the Cre/Lox System

In the protocols described in this example, human OEG cells were immortalised using a recombinant lentivirus containing the gene encoding SV40Tag flanked by Loxp sites. Cells were characterized before and after treatment with a recombinant adenovirus capable of transferring the gene encoding the Cre-recombinase to determine whether this approach could produce an OEG cell line that would be useful clinically for transplantation.

Human cells were obtained from *post-mortem* human tissue from adult donors. The tissue belongs to male and female adults donors, from the olfactory bulb; the external layer was dissociated and digested with 0.1% trypsin. The donor's age appears to be no limitant for the success of the culture. The tissue was washed with sterile phosphate-buffered saline (PBS) solution containing antibiotic (penicillin/streptomycin) and anti-fungi (Primocin). Then, the tissue was subjected to controlled proteolysis with a trypsin solution at 37°C for 20 min. After this time, the tissue was mechanically disrupted by passing the digested material through Pasteur pipettes of decreasing diameters and cultured in a specifically designed medium for this cell type. This semi-defined medium contained extract from bovine pituitary, forskolin, low amount of foetal calf serum heat-inactivated in combination with Dulbeco's modified medium (DMEM) as described in Moreno-Flores MT, Lim F, Martin-Bermejo MJ, Diaz-Nido J, Avila J, Wandosell F. "Immortalised olfactory ensheathing glia promote axonal regeneration of rat retinal ganglion neurons". J. Neurochem. 2003; 85:861-871.

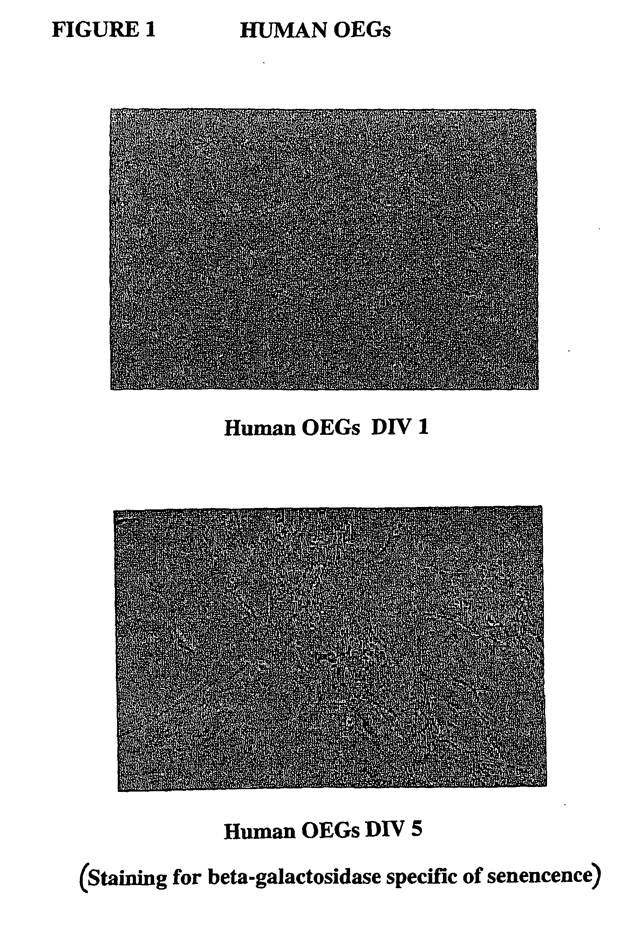

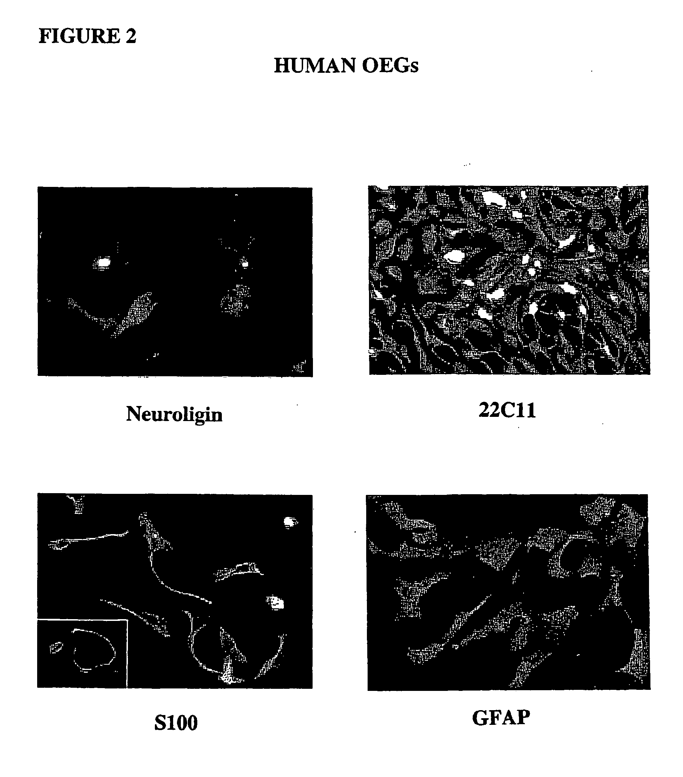

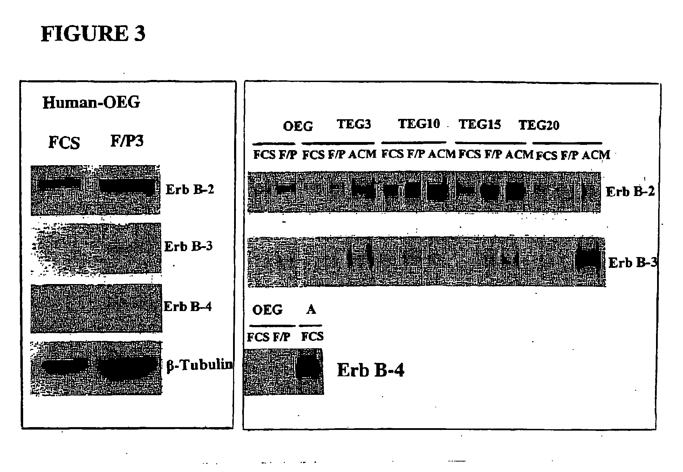

The human cells were maintained in said medium at 37°C in 7% CO₂, and then were characterized as OEG cells following the general criteria indicated in Ramon-Cueto A, Avila J. "Olfactory ensheathing glia: properties and function". Brain Res. Bull. 1998; 46:175-187. The results are shown in Figures 1-3. Table 1 summarises the different cell markers analysed from murine OEG cells, astrocytes, Schwann cells and oligodendocite-O2 astrocyte lineage and human OEG cells.

**Table 1**

| **Analysed cell markers** | | | | | | |
|---|---|---|---|---|---|---|
| Marker | Astrocyte-1 | O-2A | Schwann | mGE | | hGE |
| | | | | T.A. | T.S. | |
| O4 | - | + | + | + - | + - | n.d. |
| p75 NGFr | - | - | + | - | + | + |
| PSA-N-CAM - | | - | - | + | - | n.d. |
| GFAP | +(f) | - + | +(d) | +(f) | +/-(d) | +(d) |
| GalC | - | + | + | - | - | n.d. |
| HNK-1 | - | + | + | - | - | n.d. |
| A2B5 | - | + | - | - | - | n.d. |
| GD3 | -/+ | + | - | - | - | n.d. |
| S100 | ++ | n.d. | + | ++ | | ++ |
| 22C11 | + | n.d. | + | ++ | | ++ |
| Vim | + | n.d. | + | + | | + |
| Neuroligin | - | n.d. | n.d. | ++ | | ++ |
| Erb2 | ++ | n.d. | n.d. | ++ | | +++ |
| Erb3 | +++ | n.d. | n.d. | +/++ | | + |
| Erb 4 | +++ | n.d. | n.d. | +/- | | + |
| 3-PGDH | n.d. | n.d. | n.d. | ++ | | ++ |

| | | | | | | |
|---|---|---|---|---|---|---|
| (f) Fibrous staining ; (d): Diffuse staining; n.d.: not determined | | | | | | |

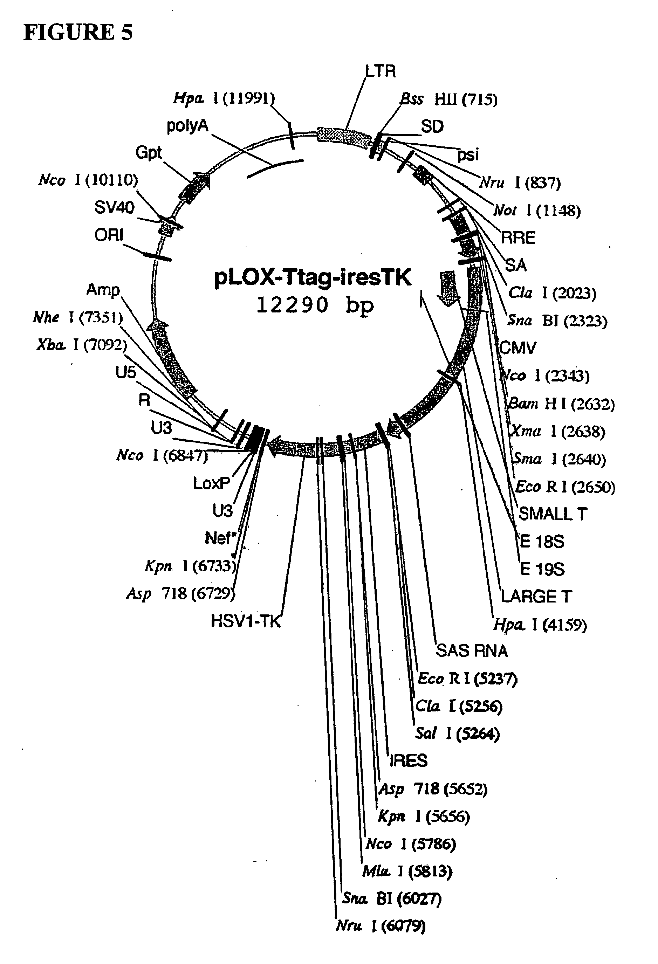

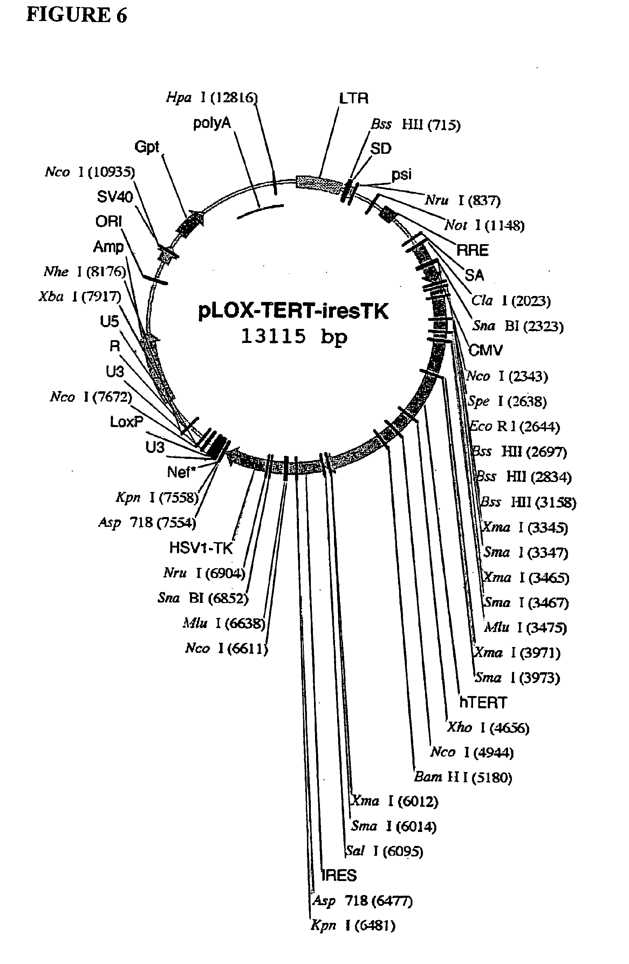

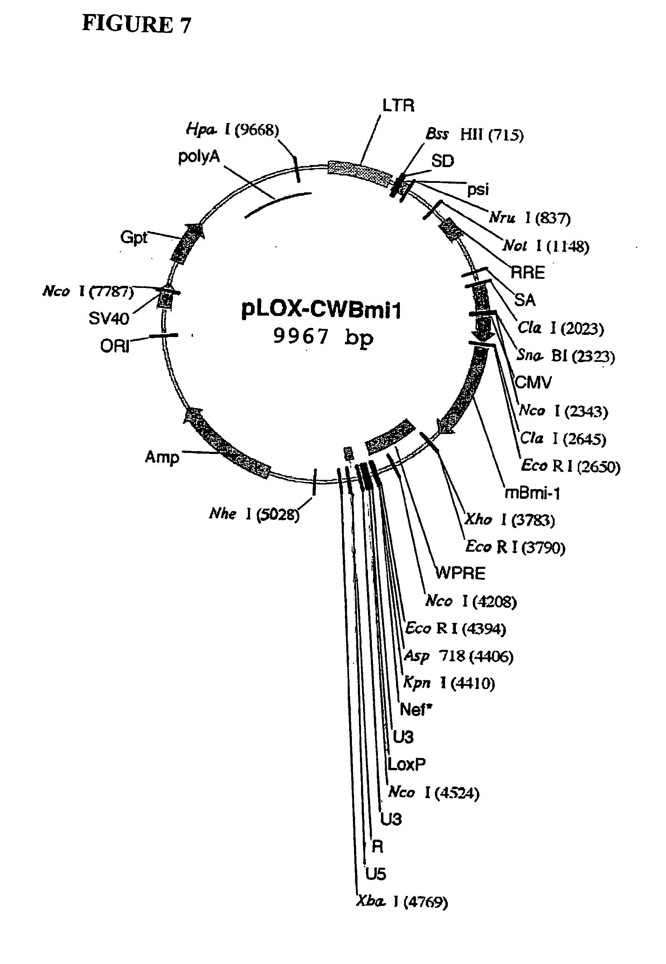

To immortalise these human OEG cells we used a reversible system, described by Dr. D. Trono's lab ( Naldini L, Blomer U, Gage FH, Trono D, Verma IM. "Efficient transfer, integration, and sustained long-term expression of the transgene in adult rat brains injected with a lentiviral vector". Proc. Natl. Acad. Sci. U.S.A 1996; 93:11382-11388; Salmon P, Oberholzer J, Occhiodoro T, Morel P, Lou J, Trono D. "Reversible immortalization of human primary cells by lentivector-mediated transfer of specific genes". Mol. Ther. 2000; 2:404-414) using the lentiviral construct denoted by pLOX-Ttag-Ires-TK (Figure 5) containing as oncogene the gene encoding SV40 large T antigen. Alternatively, the lentiviral constructs denoted by pLOX-HTERT-Ires-TK (Figure 6) containing as oncogene the gene encoding the telomerase catalytic subunit or by pLOX-CWBmil (Figure 7) containing as oncogene the gene encoding Bmi-1 protein, may also be used.

From these infections, established clones were obtained following those human cells with the highest growth rate; and initially, some clones have been obtained and
characterized as OEG cells. For instance the cells immortalised with pLOX-Ttag-Ires-TK, expressed T antigen (>85%), S100 and 3-PDGH.

### EXAMPLE 2

### Adult Retinal axonal regeneration assay

The promotion of axonal regeneration from adult rat retinal ganglion neurons (RGN) is used as a standard procedure determining regeneration capacity in cell culture. This method reflects the *in vivo* reparative properties of OEG cells, more closely than other neuritogenic assays.

Briefly, the method used was as follow: retinas were dissected from P60 rat eyes, dissociated and digested with 0.1% trypsin. Digestion was stopped with 2 mg/ml of soybean inhibitor of trypsin, and a fine suspension of cells was obtained by passing the digested material through Pasteur pipettes of decreasing diameters. Cells were centrifuged and resuspended in a defined medium (Moreno-Flores et al., 2003). RGN were plated on monolayers of glial cells: primary OEG or in the established cell lines.

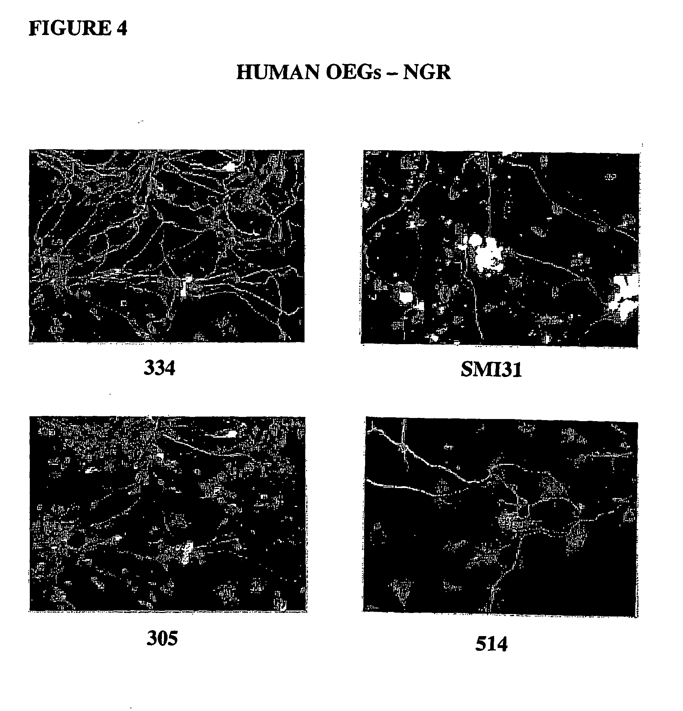

For immunocytochemistry, neuronal- human OEG cells mixed culture were fixed with 4% paraformaldehyde, after seven days of culture. Figure 4 shows the axonal regeneration capacity of rat retinal neurons stained with antibodies against neuronal proteins such as neuronal specific tubulin (334), axonal proteins such as MAP1B-SMI31 or MAP2 (305), o somato-dendritic protein MAP2 (514).

Our data show that reversibly-immortalised human OEG cells promoted axonal regeneration from adult RGN to a similar extent than either primary OEG cells or clonal cell lines from rat OEG cells.

Considering that several recent studies have demonstrated the capacity of OEG cells to promote *in vivo* CNS axonal regeneration (Li Y, Field PM, Raisman G. "Repair of adult rat corticospinal tract by transplants of olfactory ensheathing cells". Science 1997; 277:2000-2002; Li Y, Field PM, Raisman G. "Regeneration of adult rat corticospinal axons induced by transplanted olfactory ensheathing cells". J Neurosci. 1998; 18:10514-10524; Ramon-Cueto A, Plant GW, Avila J, Bunge MB. "Long-distance axonal regeneration in the transected adult rat spinal cord is promoted by olfactory ensheathing glia transplants". J.Neurosci. 1998;18:3803-3815; and Ramon-Cueto A, Cordero MI, Santos-Benito FF, Avila J. "Functional recovery of paraplegic rats and motor axon regeneration in their spinal cords by olfactory ensheathing glia". Neuron 2000; 25:425-435), with impressive motor and sensory functional recovery even after complete transection of the spinal cord [Ramon-Cueto *et al.* 2000 cited supra] and that our recent preliminary experiments show that established cell lines from rat source maintained such neuronal regenerative capacities in culture and *in vivo,* we conclude that the reversible immortalization of human OEG cells this did not produce a significant reduction in the regenerative capacity, even though the molecular mechanism underlined are not known so far.

To our knowledge this is the first time that it has been demonstrated that reversibly-immortalised human OEG cells are able to promote axonal regeneration of cultured neurons from mature mammalian CNS. Our human OEG cell lines represent useful tools to advance in the use of OEG cells in both research and applications. These cell lines and similar ones, alone or in pharmaceutical compositions comprising the same, may be used to treat CNS lesions.

## Claims

1. A method of making a population of reverse-immortalised human olfactory ensheathing glia (OEG) cells, which have the ability to promote axonal regeneration from adult CNS neurons, for transplantation into a patient, which comprises:
a) providing a sample obtained from primary human OEG cells;
b) immortalizing the OEG cells by transforming the OEG cells with a DNA construct comprising a removable DNA segment containing an oncogene or combination of oncogenes, thereby producing immortalised OEG cells;
c) growing the immortalised OEG cells;
d) selecting those immortalised OEG clonal cell lines which maintain the property of promoting axonal regeneration from adult CNS neurons; and
e) removing the oncogene or combination of oncogenes from the immortalised OEG cells, the removal resulting in the production of the population of human OEG cells for transplantation into the patient.

2. The method of claim 1, wherein the oncogene or combination of oncogenes is made removable by flanking it with recombinase target sites, and the removing is accomplished by introducing into the immortalised cells a gene that is expressed to produce a recombinase that specifically recognizes the recombinase target sites.

3. The method of claim 2, wherein the recombinase is Cre recombinase and the recombinase target sites are loxP sites.

4. The method of claim 1, wherein the oncogene is the gene encoding SV40 large T antigen, the gene encoding telomerase catalytic subunit, the gene encoding Bmi-1 protein or any combination of said oncogenes.

5. The method of claim 1, wherein the removable DNA segment further contains a suicide gene, which encodes a gene product that enables destruction of the immortalised cells by an exogenous agent if the removable DNA segment is not removed from the cells.

6. The method of claim 5, wherein the suicide gene is a gene encoding herpes simplex virus thymidine kinase, and the cells are destroyed by exposure to gancyclovir if the removable DNA segment is not removed from the cells.

7. A population of reverse-immortalised human OEG cells, which have the ability to promote axonal regeneration, producible by the method of claim 1.

8. A population of reverse-immortalised human OEG cells, which have the ability to promote axonal regeneration, producible by the method of claim 1 for use as a medicament.

9. The use of a population of reverse-immortalised human OEG cells, which have the ability to promote axonal regeneration, producible by the method of claim 1 for the manufacture of a medicament for the treatment of neural damage wherein the medicament comprises a sufficient quantity of the human OEG cells of claim 7 for transplanting into the patient to provide promotion of neuronal regeneration in the neuronal injured region of the patient.

10. A method of making a population of reverse-immortalised human OEG cells, which have the ability to promote axonal regeneration from adult CNS neurons, for transplanting into a patient, which comprises:
a) providing a sample obtained from primary human OEG cells;
b) immortalizing the OEG cells by transforming the OEG cells with a DNA construct comprising a removable DNA segment containing an oncogene or a combination of oncogenes, a selectable marker gene, and a gene encoding herpes simplex virus thymidine kinase, the genes together being flanked on either side by loxP sites;
c) growing the immortalised human OEG cells;
d) selecting those immortalised OEG clonal cell lines which maintain the property of promoting axonal regeneration from adult CNS neurons; and
e) reversing the immortalization of the human OEG cells by removing the DNA segment from the immortalised OEG cells, the removing being accomplished by introducing into the immortalised OEG cells a gene encoding Cre recombinase to effect excision of the DNA segment at the loxP sites, the excision resulting in the production of the population of human OEG cells for transplanting into a patient.

11. The method of claim 10, wherein the oncogene is the gene encoding SV40 large T antigen, the gene encoding telomerase catalytic subunit, the gene encoding Bmi-1 protein or any combination of said oncogenes.

12. A population of reverse-immortalised human OEG cells, which have the ability to promote axonal regeneration from adult CNS neurons, producible by the method of claim 10.

13. A population of reverse-immortalised human OEG cells, which have the ability to promote axonal regeneration from adult CNS neurons, producible by the method of claim 10 for use as a medicament.

14. The use of a population of reverse-immortalised human OEG cells, which have the ability to promote axonal regeneration from adult CNS neurons, producible by the method of claim 10 in the manufacture of a medicament for the treatment of neural damage wherein the medicament comprises a sufficient quantity of the OEG cells of claim 12 for transplanting into the patient to provide promotion of neuronal regeneration in the neuronal injured region.

15. A reverse-immortalised human OEG cell, which has the ability to promote axonal regeneration from adult CNS neurons, comprising a primary human OEG cell transformed with a DNA construct comprising two recombinase target sites that flank an oncogene or combination of oncogenes which confers immortalization to the OEG cell, wherein the immortalization is reversible by excision of the DNA construct by cleavage at the recombinase target sites when the target sites are exposed to a recombinase that specifically recognizes the target sites.

16. The reverse-immortalised OEG cell of claim 15, wherein the recombinase target sites are loxP sites and the immortalization is reversible by Cre recombinase cleavage at the loxP sites.

17. The reverse-immortalised OEG cell of claim 15, wherein the DNA construct further comprises a selectable marker gene.

18. The reverse-immortalised OEG cell of claim 15, wherein the DNA construct further comprises a suicide gene, which encodes a gene product that enables destruction of the immortalised OEG cell by an exogenous agent if the oncogene is not removed from the cells.

19. The reverse-immortalised OEG cell of claim 18, wherein the suicide gene is a gene encoding herpes simplex virus thymidine kinase, and the exogenous agent is gancyclovir.

20. A cell line comprising a population of the reverse-immortalised human OEG cells of claim 15.

21. A reverse-immortalised human OEG cell which has the ability to promote axonal regeneration from adult CNS neurons upon transplantation into a patient, producible by exposing the DNA construct within the immortalised human OEG cell of claim 15 to a recombinase that excises the DNA construct by cleavage at the recombinase target sites.

22. A reverse-immortalised human OEG cell which has the ability to promote axonal regeneration from adult CNS neurons upon transplantation into a patient, producible by exposing the DNA construct within the immortalised human OEG cell of claim 15 to a recombinase that excises the DNA construct by cleavage at the recombinase target sites for use as a medicament.

23. The use of a reverse-immortalised human OEG cell which has the ability to promote axonal regeneration from adult CNS neurons upon transplantation into a patient, producible by exposing the DNA construct within the immortalised human OEG cell of claim 15 to a recombinase that excises the DNA construct by cleavage at the recombinase target sites in the manufacture of a medicament for the treatment of neural damage, wherein the medicament comprises a sufficient quantity of the reverse-immortalised OEG human cells of claim 21 for transplanting into the patient to provide promotion of neuronal regeneration in the neuronal injured region of the patient.

24. A cell library comprising a population of reverse-immortalised human OEG cells which have the ability to promote axonal regeneration from adult CNS neurons, producible according to the methods of any one of claims 1 to 6 or 10 to 11.

25. A reverse immortalised human olfactory ensheathing glia (OEG) cell line, which has the ability to promote axonal regeneration from adult CNS neurons comprising a primary human OEG cell transformed with a DNA construct comprising two recombinase target sites that flank an oncogene or combination of oncogenes which confers immortalization to the OEG cell, wherein the immortalization is reversible by excision of the DNA construct by cleavage at the recombinase target sites when the target sites are exposed to a recombinase that specifically recognizes the target sites.

26. A cell line according to claim 25, for use in a method of therapy.

27. A cell line according to claim 25, for use in promoting neuronal regeneration.

28. A pharmaceutical composition comprising a human OEG cell line as defined in claims 25-27, and a pharmaceutically acceptable carrier.

29. The use of reverse-immortalised human olfactory ensheathing glia cells which have the ability to promote axonal regeneration from adult CNS neurons as defined in claims 7, 13, 22 or 25 in the preparation of a medicament for treating neuronal damage.

## Patentansprüche

1. Verfahren zum Herstellen einer Population von reversibel immortalisierten, olfaktorischen, einhüllenden Human-Gliazellen (OEG), die über die Fähigkeit verfügen, die Axon-Regeneration von Erwachsenen-CNS-Neuronen für die Transplantation in einen Patient zu fördern, welches Verfahren umfasst:
a) Bereitstellen einer von primären Human-OEG-Zellen erhaltenen Probe;
b) Immortalisieren der OEG-Zellen durch Transformieren der OEG-Zellen mit einem DNA-Konstrukt, das ein entfernbares DNA-Segment aufweist, welches ein Onkogen oder eine Kombination von Onkogenen enthält, wodurch immortalisierte OEG-Zellen erzeugt werden;
c) Aufziehen der immortalisierten OEG-Zellen;
d) Auswählen solcher immortalisierten klonalen OEG-Zelllinien, die die Eigenschaft des Förderns der Axon-Regeneration von Erwachsenen-CNS-Neuronen bewahren, und
e) Entfernen des Onkogens oder einer Kombination von Onkogenen aus den immortalisierten OEG-Zellen, wobei die Entfernung zur Erzeugung der Population von Human-OEG-Zellen für die Transplantation in den Patienten führt.

2. Verfahren nach Anspruch 1, wobei das Onkogen oder eine Kombination von Onkogenen entfernbar erzeugt wird, indem es mit Rekombinase-Targatstellen flankiert wird und das Entfernen durch Einführen in die immortalisierten Zellen eines Gens erzielt wird, das exprimiert wird, um eine Rekombinase zu erzeugen, die speziell die Rekombinase-Targetstellen erkennt.

3. Verfahren nach Anspruch 2, wobei die Rekombinase Cre-Rekombinase ist und die Rekombinase-Targetstcllen loxP-Stellen sind.

4. Verfahren nach Anspruch 1, wobei das Onkogen das Gen ist, welches SV40-groß-T-Antigen codiert, das Gen, das eine katalytische Telomerase-Subeinheit codiert, das Gen, das Bmi-1-Protein oder irgendeine Kombination dieser Onkogene codiert.

5. Verfahren nach Anspruch 1, wobei das entfernbare DNA-Segment ferner ein Suizidgen enthält, das ein Genprodukt codiert, welches die Zerstörung der immortalisierten Zellen durch ein exogenes Mittel ermöglicht, wenn das entfernbare DNA-Segment nicht aus den Zellen entfernt ist.

6. Verfahren nach Anspruch 5, wobei das Suizidgen ein Gen ist, das Herpes-simplex-Virus-Thymidinkinase codiert und die Zellen durch Exponierung an Gancyclovir exponiert wird, wenn das entfernbare DNA-Segment nicht aus den Zellen entfernt ist.

7. Population reversibel immortalisierter Human-OEG-Zellen, die über die Fähigkeit verfügen, die Axon-Regeneration zu fördern, herstellbar mit Hilfe des Verfahrens nach Anspruch 1.

8. Population von reversibel immortalisierten Human-OEG-Zellen, die über die Fähigkeit verfügen, die Axon-Regeneration zu fördern, herstellbar mit Hilfe des Verfahrens nach Anspruch 1, zur Verwendung als ein Medikament.

9. Verwendung einer Population von reversibel immortalisierten Human-OEG-Zellen, die über die Fähigkeit verfügen, die Axon-Regeneration zu fördern, herstellbar mit Hilfe des Verfahrens nach Anspruch 1 für die Herstellung eines Medikaments zur Behandlung neuraler Schädigung, wobei das Medikament eine ausreichende Menge der Human-OEG-Zellen nach Anspruch 7 zum Transplantieren in den Patienten aufweist, um eine Förderung der neuronalen Regeneration in der neuronal geschädigten Region des Patienten zu gewähren.

10. Verfahren zum Herstellen einer Population von reversibel immortalisierten Human-OEG-Zellen, die über die Fähigkeit verfügen, die Axon-Regeneration von Erwachsenen-CNS-Neuronen zum Transplantieren in einen Patienten zu fördern, umfassend:
a) Bereitstellen einer von primärem Human-OEG-Zellen erhaltenen Probe;
b) Immortalisierende OEG-Zellen durch Transformieren der OEG-Zellen mit einem DNA-Konstrukt, aufweisend ein entfernbares DNA-Segment, das ein Onkogen oder Kombination von Onkogenen, ein answählbares Markgen, und ein Herpes-simplex-Virus-Thymidinkinase codierendes Gen enthält, wobei die Gene durch die loxP-Stellen an einer oder an der anderen Seite zusammen flankiert werden;
c) Aufziehen der immortalisierten OEG-Zellen;
d) Auswählen solcher immortalisierten klonalen OEG-Zelllinien, die die Eigenschaft des Förderns der Axon-Regeneration von Erwachsenen-CNS-Neuronen bewahren, und
e) Umkehren der Immortalisierung der Human-OEG-Zellen durch Entfernen des DNA-Segmentes aus den immortalisierten OEG-Zellen, wobei das Entfernen durch Einführen eines Gens in die immortalisierten OEG-Zellen erzielt wird, das Cre-Rekombinase codiert, um eine Exzision des DNA-Segmentes an den loxP- Stellen herbeizuführen, wobei die Exzision zur Erzeugung der Population von Human-OEG-Zellen zum Transplantieren in einen Patienten führt.

11. Verfahren nach Anspruch 10, wobei das Onkogen das Gen ist, das SV40-T-Antigen codiert, das Gen ist, das telomerase-katalytische Untereinheit codiert, das Gen ist, das Bmi-1-Protein codiert oder irgendeine Kombination dieser Onkogene.

12. Population von reversibel immortalisierten Human-OEG-Zellen, die über die Fähigkeit verfügen die Axon-Regeneration von Erwachsenen-CNS-Neuronen zu fördern, herstellbar mit Hilfe des Verfahrens nach Anspruch 10.

13. Population reversibel immortalisierter Human-OEG-Zellen, die über die Fähigkeit verfügen, die Axon-Regeneration von Erwachsenen-CNS-Neuronen zu fördern, herstellbar mit Hilfe des Verfahrens nach Anspruch 10 zur Verwendung als ein Medikament.

14. Verwendung einer Population von reversibel immortalisierten Human-OEG-Zellen, die über die Fähigkeit verfügen, die Axon-Regeneration von Erwachsenen-CNS-Neuronen zu fördern, herstellbar mit Hilfe des Verfahrens nach Anspruch 10 in der Herstellung eines Medikaments für die Behandlung neuraler Schädigung, wobei das Medikament eine ausreichende Menge der OEG-Zellen nach Anspruch 12 zum Transplantieren in den Patienten aufweist, um die Förderung der neuronalen Regeneration in der neuronal geschädigten Region vorzusehen.

15. Reversibel immortalisierte Human-OEG-Zelle, die über die Fähigkeit verfügt, die Axon-Regeneration von Ezwachsenan-CNS-Neuronen zu fördern, aufweisend eine primäre Human-OEG-Zelle, die mit einem DNA-Konstrukt transformiert ist, aufweisend zwei Rekombinase-Targetstellen, die ein Onkogen oder eine Kombination von Onkogenen flankieren, die der OEG-Zelle die Immortalisierung vermitteln, wobei die Immortalisierung durch Exzision des DNA-Konstruktes durch Aufspaltung an den Rekombinase-Targetstellen reversibel ist, wenn die Targetstellen an einer Rekombinase exponiert werden, die speziell diese Targetstellen erkennt.

16. Reversibel immortalisierte OEG-Zelle nach Anspruch 15, wobei die Rekombinase-Targetstellen loxP-Stellen sind und die Immortalisierung reversibel ist durch Cre-Rekombinase Aufspaltung an den loxP-Stellen.

17. Reversibel immortalisierte OEG-Zelle nach Anspruch 15, wobei das DNA-Konstrukt ferner ein auswählbares Markergen aufweist.

18. Reversibel immortalisierte OEG-Zelle nach Anspruch 15, wobei das DNA-Konstrukt ferner ein Suizidgen aufweist, das ein Genprodukt codiert, welches die Zerstörung der immortalisierten OEG-Zelle durch ein exogenes Mittel ermöglicht, wenn das Onkogen nicht aus den Zellen entfernt ist.

19. Reversibel immortalisierte OEG-Zelle nach Anspruch 18, wobei das Suizidgen ein Gen ist, das Herpes-simplex-Virus-Thymidinkinase codiert und das exogene Mittel Gancyclovir ist.

20. Zelllinie, aufweisend eine Population der reversibel immortalisierten Human-OEG-Zellen nach Anspruch 15,

21. Reversibel immortalisierte Human-OEG-Zelle, die über die Fähigkeit verfügt, die Axon-Regeneration von Erwachsenen-CNS-Neuronen bei Transplantation in einen Patienten zu fördern, herstellbar durch Exponieren des DNA-Konstruktes im Inneren der immortalisierten Human-OEG-Zelle nach Anspruch 15 an einer Rekombinase, die das DNA-Konstrukt durch Aufspaltung an den Rokombinase-Targetstellen exzidiert.

22. Reversibel immortalisierte Human-OEG-Zelle, die über die Fähigkeit verfügt, die Axon-Regeneration von Etwachsenen-CNS-Ncuronen bei Transplantation in einen Patienten zu fördern, herstellbar durch Exponieren des DNA-Konstruktes im Inneren der immortalisierten Humän-OEQ-Zelle nach Anspruch 15 an einer Rekombinase, die das DNA-Konstrukt durch Aufspaltung an den Rckombinase-Targetstellen exzidiert, zur Verwendung als ein Medikament.

23. Verwendung einer reversibel immortalisierten Human-OEG-Zelle mit der Fähigkeit zu Förderung der Axon-Regeneration von Erwachscnen-CNS-Nauronen bei Transplantation in einen Patienten, herstellbar durch Exponieren des DNA-Konstruktes im Inneren der immortalisierten Human-OEG-Zelle nach Anspruch 15 an einer Rekombinase, die das DNA-Konstcukt durch Aufspaltung an den Rekombinase-Targetstellen exzidiert, in der Herstellung eines Medikamentes für die Behandlung neuraler Schädigung, wobei das Medikament eine ausreichende Menge an reversibel immortalisierten OEG-Human-Zellen nach Anspruch 21 aufweist, zum Transplantieren in den Patienten, um die Förderung der neuronalen Regeneration in der neuronal geschädigten Region des Patienten vorzusehen,

24. Zellenbank, aufweisend eine Population von reversibel immortalisierten Human-OEG-Zellen, die über die Fähigkeit zur Förderung der Axon-Regeneration von Erwachsenen-CNS-Neuronen verfügt, herstellbar nach den Verfahren nach einem der Ansprüche 1 bis 6 oder 10 und 11.

25. Reversibel immortalisierte, olfaktorische, einhüllende Glia(OEG)-Zelllinie, die über die Fähigkeit zur Förderung der Axon-Regeneration von Erwachsenen-CNS-Neuronen verfügt, aufweisend eine primäre Human-OEG-Zelle, transformiert mit einem DNA-Konstrukt, aufweisend zwei Rekombinase-Targetstellen, die ein Onkogen oder eine Kombination von Onkogenen flankiert, die der OEG-Zelle die Immortalisation vermittelt, wobei die Immortalisation reversibel ist durch Exzision des DNA-Konstruktes durch Aufspaltung an den Rekombinase-Targetstellen, wenn die Targetstellen an einer Rekombinase exponiert sind, die speziell diese Targetstellen erkennt.

26. Zelllinien nach Anspruch 25 für die Verwendung in einem Verfahren für Therapie.

27. Zelllinien nach Anspruch 25 zur Verwendung für die Förderung der neuronalen Regeneration.

28. Pharmazeutische Zusammensetzung, aufweisend eine Human-OEG-Zelllinie nach Anspruch 25 bis 27, und einen pharmazeutisch duldbaren Träger.

29. Verwendung von reversibel immortalisierten, olfaktorischen, einhüllenden Human-Glia-Zellen, die über die Fähigkeit zur Förderung der Axon-Regeneration von Erwachsenen-CNS-Neuronen nach Anspruch 7, 13, 22 oder 25 verfügen, in der Herstellung eines Medikamentes zur Behandlung einer neuronalen Schädigung.

## Revendications

1. Procédé de préparation d'une population de cellules gliales engainantes olfactives (OEG) humaines à immortalisation inversée, qui ont l'aptitude à stimuler la régénération axonale à partir de neurones adultes du SNC, pour une transplantation dans un patient, qui comprend:
a) la fourniture d'un échantillon obtenu à partir de cellules OEG humaines primaires;
b) l'immortalisation des cellules OEG en transformant les cellules OEG avec un produit d'assemblage d'ADN comprenant un segment d'ADN amovible contenant un oncogène ou une combinaison d'oncogènes, produisant ainsi des cellules OEG immortalisées;
c) la croissance des cellules OEG immortalisées;
d) la sélection des lignées de cellules OEG clonales immortalisées qui maintiennent la propriété de stimulation de la régénération axonale à partir de neurones adultes du SNC; et
e) l'enlèvement de l'oncogène ou de la combinaison d'oncogènes à partir des cellules OEG immortalisées, l'enlèvement conduisant à la production de la population de cellules OEG humaines pour une transplantation dans le patient.

2. Procédé selon la revendication 1, dans lequel l'oncogène ou la combinaison d'oncogènes est rendu(e) amovible en le/la flanquant avec des sites cibles de recombinase, et l'enlèvement est accompli en introduisant dans les cellules immortalisées un gène qui est exprimé pour produire une recombinase qui reconnaît spécifiquement les sites cibles de recombinase.

3. Procédé selon la revendication 2, dans lequel la recombinase est la Cre recombinase et les sites cibles de recombinase sont des sites loxP.

4. Procédé selon la revendication 1, dans lequel l'oncogène est le gène codant le gros antigène T de SV40, le gène codant une sous-unité catalytique de la télomérase, le gène codant la protéine Bmi-1 ou toute combinaison desdits oncogènes.

5. Procédé selon la revendication 1, dans lequel le segment d'ADN amovible contient en outre un gène suicide, qui code un produit génique qui permet la destruction des cellules immortalisées par un agent exogène si le segment d'ADN amovible n'est pas enlevé des cellules.

6. Procédé selon la revendication 5, dans lequel le gène suicide est un gène codant la thymidine kinase de virus herpès simplex, et les cellules sont détruites par exposition à du gancyclovir si le segment d'ADN amovible n'est pas enlevé des cellules.

7. Population de cellules OEG humaines à immortalisation inversée, qui ont l'aptitude à stimuler la régénération axonale, productibles par le procédé selon la revendication 1.

8. Population de cellules OEG humaines à immortalisation inversée, qui ont l'aptitude à stimuler la régénération axonale, productibles par le procédé selon la revendication 1, pour l'utilisation en tant que médicament.

9. Utilisation d'une population de cellules OEG humaines à immortalisation inversée, qui ont l'aptitude à stimuler la régénération axonale, productibles par le procédé selon la revendication 1, pour la fabrication d'un médicament pour le traitement d'une lésion neurale, dans laquelle le médicament comprend une quantité suffisante des cellules OEG humaines selon la revendication 7 pour une transplantation dans le patient pour permettre une stimulation de la régénération neuronale dans la région du patient à lésion neuronale.

10. Procédé de préparation d'une population de cellules OEG humaines à immortalisation inversée, qui ont l'aptitude à stimuler la régénération axonale à partir de neurones adultes du SNC, pour une transplantation dans un patient, qui comprend:
a) la fourniture d'un échantillon obtenu à partir de cellules OEG humaines primaires;
b) l'immortalisation des cellules OEG en transformant les cellules OEG avec un produit d'assemblage d'ADN comprenant un segment d'ADN amovible contenant un oncogène ou une combinaison d'oncogènes, un gène marqueur sélectionnable, et un gène codant la thymidine kinase de virus herpès simplex, les gènes étant ensemble flanqués sur l'un ou l'autre côté par des sites loxP;
c) la croissance des cellules OEG humaines immortalisées;
d) la sélection des lignées de cellules OEG clonales immortalisées qui maintiennent la propriété de stimulation de la régénération axonale à partir de neurones adultes du SNC; et
e) l'inversion de l'immortalisation des cellules OEG humaines en enlevant le segment d'ADN à partir des cellules OEG immortalisées, l'enlèvement étant accompli en introduisant dans les cellules OEG immortalisées un gène codant la Cre recombinase pour effectuer l'excision du segment d'ADN aux sites loxP, l'excision conduisant à la production de la population de cellules OEG humaines pour une transplantation dans un patient.

11. Procédé selon la revendication 10, dans lequel l'oncogène est le gène codant le gros antigène T de SV40, le gène codant la sous-unité catalytique de la télomérase, le gène codant la protéine Bmi-1 ou toute combinaison desdits oncogènes.

12. Population de cellules OEG humaines à immortalisation inversée, qui ont l'aptitude à stimuler la régénération axonale à partir de neurones adultes du SNC, productibles par le procédé selon la revendication 10.

13. Population de cellules OEG humaines à immortalisation inversée, qui ont l'aptitude à stimuler la régénération axonale à partir de neurones adultes du SNC, productibles par le procédé selon la revendication 10, pour l'utilisation en tant que médicament.

14. Utilisation d'une population de cellules OEG humaines à immortalisation inversée, qui ont l'aptitude à stimuler la régénération axonale à partir de neurones adultes du SNC, productibles par le procédé selon la revendication 10, dans la fabrication d'un médicament pour le traitement d'une lésion neurale, dans laquelle le médicament comprend une quantité suffisante des cellules OEG selon la revendication 12 pour une transplantation dans le patient pour prévoir la stimulation de la régénération neuronale dans la région à lésion neuronale.

15. Cellule OEG humaine à immortalisation inversée, qui a l'aptitude à stimuler la régénération axonale à partir de neurones adultes du SNC, comprenant une cellule OEG humaine primaire transformée avec un produit d'assemblage d'ADN comprenant deux sites cibles de recombinase qui flanquent un oncogène ou une combinaison d'oncogènes qui confère une immortalisation à la cellule OEG, dans laquelle l'immortalisation est réversible par excision du produit d'assemblage d'ADN par clivage aux sites cibles de recombinase lorsque les sites cibles sont exposés à une recombinase qui reconnaît spécifiquement les sites cibles.

16. Cellule OEG à immortalisation inversée selon la revendication 15, dans laquelle les sites cibles de recombinase sont des sites loxP et l'immortalisation est réversible par clivage aux sites loxP par la Cre recombinase.

17. Cellule OEG à immortalisation inversée selon la revendication 15, dans laquelle le produit d'assemblage d'ADN comprend en outre un gène marqueur sélectionnable.

18. Cellule OEG à immortalisation inversée selon la revendication 15, dans laquelle le produit d'assemblage d'ADN comprend un outre un gène suicide, qui code un produit génique qui permet la destruction de la cellule OEG immortalisée par un agent exogène si l'oncogène n'est pas enlevé des cellules.

19. Cellule OEG à immortalisation inversée selon la revendication 18, dans laquelle le gène suicide est un gène codant la thymidine kinase de virus herpès simplex, et l'agent exogène est le gancyclovir.

20. Lignée cellulaire comprenant une population des cellules OEG humaines à immortalisation inversée selon la revendication 15.

21. Cellule OEG humaine à immortalisation inversée, qui a l'aptitude à stimuler la régénération axonale à partir de neurones adultes du SNC par transplantation dans un patient, productible en exposant le produit d'assemblage d'ADN à l'intérieur de la cellule OEG humaine immortalisée selon la revendication 15 à une recombinase qui excise le produit d'assemblage d'ADN par clivage aux sites cibles de recombinase.

22. Cellule OEG humaine à immortalisation inversée, qui a l'aptitude à stimuler la régénération axonale à partir de neurones adultes du SNC par transplantation dans un patient, productible en exposant le produit d'assemblage d'ADN à l'intérieur de la cellule OEG humaine immortalisée selon la revendication 15 à une recombinase qui excise le produit d'assemblage d'ADN par clivage aux sites cibles do recombinase, pour l'utilisation en tant que médicament.

23. Utilisation d'une cellule OEG humaine à immortalisation inversée, qui a l'aptitude à stimuler la régénération axonale à partir de neurones adultes du SNC par transplantation dans un patient, productible en exposant le produit d'assemblage d'ADN à l'intérieur de la cellule OEG humaine immortalisée selon la revendication 15 à une recombinase qui excise le produit d'assemblage d'ADN par clivage aux sites cibles de recombinase, dans la fabrication d'un médicament pour le traitement d'une lésion neurale, dans laquelle le médicament comprend une quantité suffisante des cellules OEG humaines à immortalisation inversée selon la revendication 21 pour une transplantation dans le patient pour prévoir la stimulation de la régénération neuronale dans la région du patient à lésion neuronale.

24. Répertoire de cellules comprenant une population de cellules OEG humaines à immortalisation inversée, qui ont l'aptitude à stimuler la régénération axonale à partir de neurones adultes du SNC, productibles d'après les procédés selon l'une quelconque des revendications 1 à 6 ou 10 à 11.

25. Lignée de cellules gliales engainantes olfactives (OEG) humaines à immortalisation inversée, qui a l'aptitude à stimuler la régénération axonale à partir de neurones adultes du SNC, comprenant une cellule OEG humaine primaire transformée avec un produit d'assemblage d'ADN comprenant deux sites cibles de recombinase qui flanquent un oncogène ou une combinaison d'oncogènes qui confère une immortalisation à la cellule OEG, dans laquelle l'immortalisation est réversible par excision du produit d'assemblage d'ADN par clivage aux sites cibles de rccombinase lorsque les sites cibles sont exposés à une recombinase qui reconnaît spécifiquement les sites cibles.

26. Lignée cellulaire selon la revendication 25, pour l'utilisation dans un procédé de thérapie.

27. Lignée cellulaire selon la revendication 25, pour l'utilisation dans la stimulation de la régénération neuronale.

28. Composition pharmaceutique comprenant une lignée cellulaire OEG humaine telle que définie dans les revendications 25 à 27, et un véhicule pharmaceutiquement acceptable.

29. Utilisation de cellules gliales engainantes olfactives humaines à immortalisation inversée qui ont l'aptitude à stimuler la régénération axonale à partir de neurones adultes du SNC telles que définies dans les revendications 7, 13, 22 ou 25, dans la préparation d'un médicament pour traiter une lésion neuronale.
